# EUROPÄISCHE PATENTSCHRIFT

(11) **EP 2 793 694 B1**
(45) Veröffentlichungstag und Bekanntmachung des Hinweises auf die Patenterteilung: **16.03.2016**
(21) Anmeldenummer: 12823141.2
(22) Anmeldetag: 20.12.2012
(51) Int. Cl.: A61B 5/04

(54) **MAGNETKARDIOGRAPHIEANORDNUNG UND ERGOMETER HIERFÜR**
MAGNETOCARDIOGRAPHY ARRANGEMENT AND ERGOMETER FOR SAME
SYSTÈME DE MAGNÉTOCARDIOGRAPHIE ET ERGOMÈTRE ASSOCIÉ

(30) Priorität: 21.12.2011 DE 202011052404 U; 21.12.2011 DE 102011056786; 22.12.2011 DE 202011052433 U; 22.12.2011 DE 102011056881
(43) Veröffentlichungstag der Anmeldung: 29.10.2014
(73) Patentinhaber: Biomagnetik Park GmbH, 21149 Hamburg (DE)
(72) Erfinder: JUNG, Friedrich, 01259 Dresden (DE); JANG, Honjoe, Seongdong-gu, Seoul (KR); SCHMIDT, Jens, 21441 Garstedt (DE); KRAUSE, Dieter, 21244 Buchholz (DE)
(74) Vertreter: Stüven, Ralf
(86) Internationale Anmeldenummer: PCT/DE2012/100394
(87) Internationale Veröffentlichungsnummer: WO 2013/091627

(56) Entgegenhaltungen:
- EP-A1- 1 714 615
- DE-A1-102006 056 282
- DE-B1- 2 630 025
- Dania Di Pietro Paolo: "Numerical methods for improved signal to noise ratios in spatiotemporal biomedical data (Dissertation)", , 4. August 2010 (2010-08-04), Seiten FP,i-xii,1-138, XP002695420, DB Thueringen Gefunden im Internet: URL:http://www.db-thueringen.de/servlets/D ocumentServlet?id=16261 [gefunden am 2013-04-15]
- QUISTORFF B ET AL: "A SIMPLE CALF MUSCLE ERGOMETER FOR USE IN A STANDARD WHOLE-BODY MR SCANNER", MAGNETIC RESONANCE IN MEDICINE, Bd. 13, Nr. 3, 1. März 1990 (1990-03-01), Seiten 444-449, XP000132474, ACADEMIC PRESS, DULUTH, MN, US ISSN: 0740-3194

## Beschreibung

Die Erfindung betrifft eine Magnetkardiographieanordnung sowie ein Ergometer hierfür.

Bei der Magnetkardiographie handelt es sich um ein Verfahren, bei dem die magnetische Aktivität des Herzens kontaktlos registriert wird. Um das vom menschlichen Herzen generierte extrem schwache Magnetfeld messen zu können, werden hochempfindliche Detektoren aus supraleitenden Quanteninterferometern, so genannte SQUIDs, eingesetzt.

Mit Hilfe der Magnetkardiographie werden zur Feststellung von Herzfehlern, z.B. einer koronaren Herzkrankheit, Belastungs-Magnetkardiogramme erstellt. Dabei wird eine Belastung des Herzens in der Regel auf pharmakologischem Wege durch Injektion eines geeigneten Mittels, z.B. Adenosin oder Dobutamin, herbeigeführt. Bekannt ist auch die Belastung mittels eines Ergometers.

Aus der EP 0371156 A1 ist beispielsweise eine Magnetkardiographieanordnung bekannt, bei der ein aus nichtmagnetischen Bauelementen bestehendes Fahrrad-Ergometer auf Schwungradbasis verwendet wird. Darüber hinaus ist aus der DE 10 2006 056 282 A1 eine Magnetkardiographieanordnung bekannt, bei der ebenfalls ein nicht magnetisches Fahrrad-Ergometer Verwendung findet, diesmal jedoch auf Basis einer Hydraulik.

Aufgabe der vorliegenden Erfindung ist es, eine verbesserte Magnetkardiographieanordnung zur Erstellung von Belastungs-Magnetkardiogrammen bereitzustellen.

Diese Aufgabe wird durch den Gegenstand des Anspruchs 1 gelöst. Zweckmäßige Ausgestaltungen der Erfindung sind in den Unteransprüchen angegeben.

Die Magnetkardiographieanordnung umfasst eine Belastungseinheit, die auf einem mittels druckbeaufschlagtem Gas oder druckbeaufschlagter Gasmischung, bevorzugt pneumatisch, gebremsten aus nicht-magnetischen Materialien bestehenden Ergometer basiert. Ein in geeigneter Weise ausgestaltetes Ergometer ist weiter unten beschrieben. Die Verwendung einer Kompressionsgasbremse, beispielsweise einer Pneumatikbremse, hat den Vorteil, dass die entsprechenden Komponenten in der Abschirmkammer relativ problemlos komplett aus nicht-magnetischen Materialien hergestellt werden können, wie beispielsweise Leitungen aus Kupfer oder Kunststoff. Probleme mit einem etwaigen Austritt von Hydraulikflüssigkeit werden ebenfalls vermieden. Dadurch, dass die Kompressionsgasquelle, z.B. die Druckluftquelle, außerhalb der Abschirmkammer angeordnet ist, wird eine weitere Störungsursache für die empfindliche Messung vermieden. Darüber hinaus ist es im Falle der Verwendung von Druckluft vorteilhaft, dass die Belastungseinheit an die in Kliniken häufig übliche zentrale Druckluftversorgung angeschlossen werden kann.

Unter einem "Ergometer" wird hier eine Vorrichtung verstanden, mit deren Hilfe eine Person einer definierten körperlichen Belastung unterzogen werden kann. Unter einem Fahrrad-Ergometer wird hier ein Ergometer verstanden, bei dem durch die Person eine Kurbel beispielsweise mittels Pedalen gegen einen Widerstand in eine beispielsweise kreisförmig Drehbewegung versetzt wird. Dabei kann die Drehbewegung durch Bewegung der Beine und/oder der Arme erzeugt werden. "Nicht-magnetisch" bedeutet, dass die Materialien kein oder kein nennenswertes die Messung durch die SQUIDs störendes Magnetfeld aufweisen, insbesondere nicht ferromagnetisch sind. Beispiele für solche Materialien sind Keramiken wie Zirkonoxid (ZrO₂), Siliciumnitrid (SiN₄), Kunststoffe wie Polyoxymethylen (POM), Polytetrafluorethylen (PTFE) oder Polyetheretherketon (PEEK), oder Metalle wie Aluminium und Kupfer. Unter einem "druckbeaufschlagten Gas" oder einer "druckbeaufschlagten Gasmischung" wird jedes Gas bzw. jede Gasmischung verstanden, die mit Druck beaufschlagt ist. Der hier verwendete Ausdruck "Kompressionsgas" ist synonym zu den Ausdrücken "druckbeaufschlagtes Gas" und "druckbeaufschlagte Gasmischung" und umfasst beide Begriffe. Bei dem(den) Gas(en) kann es sich beispielsweise um Luft, Stickstoff, Helium, Sauerstoff oder beliebige Gasmischungen, z.B. eine Stickstoff-Sauerstoff-Mischung, handeln. Es kann sich auch um ein Gas bzw. eine Gasmischung handeln, die unter Druck und/oder Kühlung verflüssigt ist, z.B. flüssigen Stickstoff. Vorzugsweise handelt es sich um Druckluft, d.h. komprimierte Umgebungsluft. Der Druck kann beispielsweise bei 5 bar, gegebenenfalls aber auch niedriger oder höher sein. Unter den Begriffen "Quelle für ein druckbeaufschlagtes Gas", "Quelle für eine druckbeaufschlagte Gasmischung" oder "Kompressiongasquelle" wird die Stelle verstanden, von der das unter Druck stehende Gas stammt oder vorgehalten wird, bevor es gegebenenfalls über entsprechende Leitungen weitergeleitet wird. Es kann sich beispielsweise um einen oder mehrere Kompressoren und/oder Druckgasflaschen handeln. Unter einem "Magnetkardiograph" wird ein Gerät zur Messung von kardiomagnetische Feldern verstanden. Bei einem kardiomagnetischen Feld handelt es sich um ein biomagnetisches Feld, dass durch die elektrische Aktivität oder Interaktion von Herzzellen, beispielsweise im menschlichen Körper, erzeugt wird. Kardiomagnetische Felder sind vergleichsweise schwach und liegen beispielsweise im Bereich von einigen zehn Pikotesla (1 pT = 10⁻¹² T).

Die Belastungseinheit ist vorzugsweise an einer aus nicht-magnetischen Komponenten bestehenden Patientenliege angeordnet, kann aber auch separat vorgesehen sein.

Besonders bevorzugt sind der Magnetkardiograph und die Belastungseinheit in einer Abschirmkammer zur Abschirmung externer Magnetfelder angeordnet, und die Belastungseinheit ist mittels nicht-magnetischer Leitungen mit einer vorzugsweise innerhalb der Abschirmkammer angeordneten Druckregelungseinheit verbunden. Die Druckregelungseinheit ist mit der außerhalb der Abschirmkammer angeordneten Quelle für ein druckbeaufschlagtes Gas oder eine druckbeaufschlagte Gasmischung, z.B. einer Druckluftquelle, verbunden. Bei der Druckluftquelle kann es sich um die zentrale Druckluftversorgung einer Klinik handeln.

Vorzugsweise sind der Magnetkardiograph und/oder die Druckregelungseinheit mit einer außerhalb der Abschirmkammer angeordneten Bedien- und Steuereinheit verbunden. Besonders bevorzugt kann der mittels der Druckregelungseinheit eingestellte oder einstellbare Widerstand für die Belastungseinheit über die Bedien- und Steuereinheit gemessen und/oder geregelt werden. Auf diese Weise kann die Belastungseinheit und damit der Patient mit einer vorgegebenen Belastung beaufschlagt werden und die anliegende Belastung kann gemessen und gegebenenfalls aufgezeichnet werden. Bei der Bedien- und Steuereinheit kann es sich um einen in geeigneter Weise eingerichteten Personal Computer handeln.

Es ist weiter bevorzugt, parallel zum Magnetkardiogramm (MGK) ein Elektrokardiogramm (EKG) aufzuzeichnen und zu diesem Zweck ein außerhalb der Abschirmkammer positioniertes EKG-Gerät vorzusehen, das aus nicht-magnetischen Komponenten bestehende, in die Abschirmkammer reichende und mit dem Patienten verbindbare EKG-Elektrodenstecker und -Ableitungen aufweist.

Um eine Patientenüberwachung zu erleichtern, ist in der Abschirmkammer vorzugsweise eine Überwachungskamera in einem magnetisch geschirmten Gehäuse vorgesehen, deren Signale über geschirmte Kabel aus der Abschirmkammer und vorzugsweise zur Bedien- und Steuereinheit geleitet werden.

Es wird auch ein Ergometer bereitgestellt, das zur Verwendung bei der Erstellung von Belastungs-Magnetkardiogrammen besonders gut geeignet ist, und das als Belastungseinheit in der erfindungsgemäßen Magnetkardiographieanordnung vorteilhaft eingesetzt werden kann. Das Ergometer besteht aus nicht-magnetischem Material und umfasst eine Schwungmasse, die mittels Hand- oder Fußbetätigung über ein Getriebe mit einem Übersetzungsverhältnis von mindestens 1:10 in Rotation versetzbar ist.

Bei dem Ergometer, das vorzugsweise ein Fahrradergometer ist, wird eine Schwungmasse über ein Getriebe in Rotation versetzt. Das Getriebe ist dabei bevorzugt mindestens zweistufig. Dadurch, dass die Schwungmasse von einem Getriebe mit einem hohen Übersetzungsverhältnis von mindestens 1:10 angetrieben wird, wird eine besonders gleichförmige und für z.B. kranke Patienten angenehme Bedienung ermöglicht. Für z.B. herzkranke Menschen ist es häufig schon nicht einfach oder sogar unmöglich, einen stark über den Drehwinkel schwankenden Widerstand, den die Bremse der Antriebsbewegung entgegensetzt, zu überwinden. Durch die mittels des hohen Übersetzungsverhältnisses erreichte vergleichsweise hohe Drehzahl der Schwungmasse kann auch bei der häufig geringen Dichte nicht-magnetischer Materialien bei gleichem Bauraum ein höheres Energiespeichervermögen erreicht werden. Ein hohes Energiespeichervermögen ermöglicht wiederum die Realisierung einer gleichförmigen Tretbewegung, Die Erfindung ermöglicht es, das Energiespeichervermögen des Ergometers so weit zu steigern, dass eine Überwindung der Totpunkte des Antriebs, z.B. des Kurbelantriebs, auch bei gebremstem Ergometer leicht möglich ist. Somit wird eine maßvolle und gezielte Belastung von Patienten möglich, um beispielsweise ein Magnetkardiogramm unter Belastung zu erstellen.

Das Übersetzungsverhältnis beträgt bevorzugt 1:10 bis 1:20, weiter bevorzugt 1:10 bis 1:18, weiter bevorzugt 1:10 bis 1:15 und besonders bevorzugt 1:10 bis 1:13, beispielsweise etwa 1:12. Auch Zwischenwerte der obigen Bereiche, z.B. 1:11, 1:12,25 und andere Verhältnisse sind hier ausdrücklich mit offenbart.

In einer bevorzugten Ausführungsform umfasst das Ergometer ferner eine mittels druckbeaufschlagtem Gas oder druckbeaufschlagter Gasmischung betätigbare Bremseinheit zur Abbremsung der Rotation der Schwungmasse. Dabei ist die Bremskraft der Bremseinheit vorzugsweise mittels des druckbeaufschlagten Gases oder der druckbeaufschlagten Gasmischung einstellbar. Bei dem druckbeaufschlagten Gas oder der druckbeaufschlagten Gasmischung kann es sich um Druckluft, Stickstoff, Sauerstoff, Helium, Sauerstoff oder Mischungen davon handeln. Besonders bevorzugt ist die pneumatische Einstellung der Bremskraft der Bremseinheit mittels Druckluft. Die Verwendung einer Kompressionsgasbremse, beispielsweise einer Pneumatikbremse, hat den Vorteil, dass die entsprechenden Komponenten, beispielsweise Leitungen, in der Abschirmkammer aus nicht-magnetischen Materialien wie beispielsweise Kupfer oder Kunststoff hergestellt werden können. Darüber hinaus ist es beispielsweise im Falle der Verwendung von Druckluft vorteilhaft, dass das Ergometer an die in Kliniken häufig übliche zentrale Druckluftversorgung angeschlossen werden kann. Vorteilhaft ist eine stufenlos einstellbare Bremskraft der Bremseinheit über eine geeignete Steuerung, so dass ein definiertes Bremsdrehmoment zur Belastung des Patienten einstellbar ist.

Bei der Bremseinheit handelt es sich vorzugsweise um einen Pneumatikzylinder, in dem ein Kolben beweglich angeordnet ist, der beispielsweise mit einer Bremsscheibe vorzugsweise kraftschlüssig in Eingriff bringbar ist, so dass über den Luftdruck die von der Bremseinheit erzeugte Bremskraft einstellbar ist. Die Bremskraft der Bremseinheit wird bevorzugt über eine Drucksteuerung eingestellt. Mittels dieser Drucksteuerung kann eine definierte und vorzugsweise kontinuierlich veränderbare Bremswirkung eingestellt werden.

In einer bevorzugten Ausgestaltung des Ergometers umfasst dieses ein zweistufiges Getriebe, das aus vier Wellen besteht, wobei zwei Wellen als Hohlwellen ausgebildet sind, die jeweils koaxial zu einer der beiden anderen vorzugsweise als Vollwellen ausgebildeten Wellen angeordnet sind. Bei einer Ausgestaltung als Fahrradergometer sind vorzugsweise an der bevorzugt als Vollwelle ausgestalteten ersten Welle Pedalarme montiert, über die die erste Welle durch Tretbewegungen in Rotation versetzt werden kann. Über eine erste Getriebestufe wird die Drehbewegung von der ersten Welle auf die ebenfalls bevorzugt als Vollwelle ausgestaltete zweite Welle, übertragen und die Drehzahl gewandelt. Über die zweite Getriebestufe wird die Drehbewegung von der als Hohlwelle ausgebildeten dritten Welle auf die ebenfalls als Hohlwelle ausgebildete vierte Welle übertragen und die Drehzahl gewandelt. Mit der vierten Welle ist die Schwungmasse form- oder kraftschlüssig verbunden. In dieser Ausführungsform ist das erfindungsgemäße Ergometer besonders kompakt, so dass es beispielsweise leichter in eine Liege integriert werden kann.

In einer besonders bevorzugten Ausführungsform ist zwischen der ersten und zweiten Getriebestufe eine Freilaufkupplung angeordnet. Hierzu kann die Freilaufkupplung beispielsweise zwischen der zweiten Welle und der auf ihr gelagerten Hohlwelle angeordnet sein. Die Integration einer Freilaufkupplung ist besonders vorteilhaft, um im Falle eines Fahrradergometers eine weitere Rotation der Tretkurbel bzw. der Pedalarme zu verhindern, wenn die das Ergometer benutzende Person langsamer tritt oder mit dem Treten aufhört. Insbesondere bei älteren und/oder geschwächten Personen ist dies aus Sicherheitsgründen vorteilhaft.

Bei dem Getriebe kann es sich beispielsweise um ein Zahnradgetriebe oder ein Riemengetriebe handeln. In einer bevorzugten Ausführungsform handelt es sich um Riemengetriebe. Im Falle eines Riemengetriebes ist vorzugsweise auf der ersten Welle ein erstes Riemenrad, auf der zweiten Welle ein zweites Riemenrad, auf der dritten Welle ein drittes Riemenrad und auf der vierten Welle ein viertes Riemenrad angeordnet, wobei das erste und zweite Riemenrad sowie das dritte und vierte Riemenrad jeweils über einen Riemen verbunden sind.

Bei einer bevorzugten Ausfuhrungsform des Ergometers ist mit der als Hohlwelle ausgebildeten vierten Welle eine Bremsscheibe form- oder kraftschlüssig verbunden, die mit einem Pneumatikzylinder der Bremseinheit in Eingriff gebracht werden kann. Auf diese Weise wird eine wirkungsvolle Bremsung der Rotation der Schwungmasse ermöglicht. Die Bremsscheibe und/oder der Kolben des Pneumatikzylinders weisen einen geeigneten Bremsbelag auf.

Die Erfindung wird im Folgenden anhand der beigefügten Figuren rein zu Veranschaulichungszwecken näher erläutert. Es zeigt
Fig. 1 ein schematisches Schaubild einer Magnetkardiographieanordnung.
Fig. 2 eine Prinzipskizze eines Ergometers,
Fig. 3 eine Darstellung einer Ausführungsform eines Ergometers in einer (A) Front- und (B) Rückansicht,
Fig. 4 eine isometrische Ansicht des Ergometers aus Fig. 3.

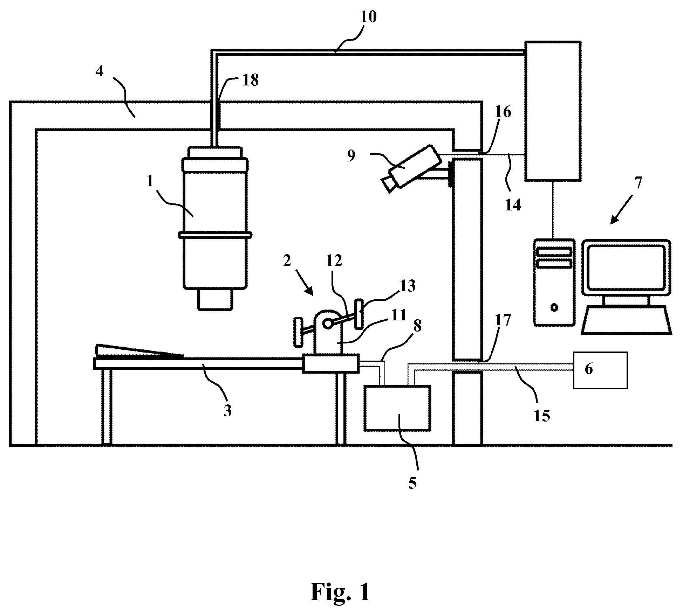

Figur 1 zeigt eine Magnetkardiographieanordnung. Gezeigt ist ein Magnetkardiograph 1 über einer Patientenliege 3, an deren Fußende eine als Fahrradergometer ausgestaltete Belastungseinheit 2 vorgesehen ist. Der Magnetkardiograph 1 und die Patientenliege 3 mit der Belastungseinheit 2 befinden sich in einer Abschirmkammer 4, deren Innenraum von externen elektromagnetischen Störeinflüssen abgeschirmt ist.

Patientenliege 3 und Belastungseinheit 2 sind komplett aus nicht-ferromagnetischen Werkstoffen, wie beispielsweise Aluminium und geeigneten Kunststoffen, gefertigt. Der Magnetkardiograph 1 bzw. dessen Sensoreinheit sind über geeignete Versorgungs- und Signalleitungen 10 mit der Bedien- und Steuereinheit 7 außerhalb der Abschirmkammer 4 verbunden.

Zur Aufnahme eines Belastungs-Magnetkardiogramms ist ein Fahrradergometer 2 in Längsrichtung der Patientenliege 3 verschiebbar am Fußende der Patientenliege 3 angeordnet. Das Fahrradergometer 2 umfasst in einem Gehäuse 11 drehbar gelagerte Pedalarme 12 mit Pedalen 13. In dem Gehäuse 11 sind Leitungen 8 für Druckluft vorgesehen, die mit einer Druckregeleinheit 5 verbunden sind. Weitere Leitungen 15 sind mit einer außerhalb der Abschirmkammer 4 liegenden Druckluftquelle 6 verbunden. Statt Druckluft kann beispielsweise auch ein verflüssigtes Gas, z.B. Stickstoffgas, in einem Druckbehälter vorgehalten werden. Bei Raumtemperatur baut sich ein entsprechender Druck auf. In diesem Fall kann die Druckregeleinheit 5 auch an dem Druckbehälter angebracht sein und die Leitungen 8 können direkt mit der Druckregeleinheit 5 verbunden sein.

Alle Teile des Fahrradergometers 2 und der Leitungen 8, die innerhalb der Abschirmkammer 4 angeordnet sind, sind aus nicht-ferromagnetischen Materialien hergestellt.

Zur Überwachung des auf der Patientenliege 3 ruhenden Patienten (hier nicht dargestellt) ist eine in einem magnetisch geschirmten Gehäuse untergebrachte Überwachungskamera 9 in der Abschirmkammer 4 montiert. Das Kamerabild wird über eine aus der Abschirmkammer 4 herausgeführte Signalleitung 14 zur Bedien- und Steuereinheit 7 oder auf einen separaten Monitor (nicht dargestellt) übertragen.

Sämtliche Versorgungs- oder Verbindungsleitungen sind über entsprechende Durchführungen 16, 17, 18 in die Abschirmkammer 4 gelegt.

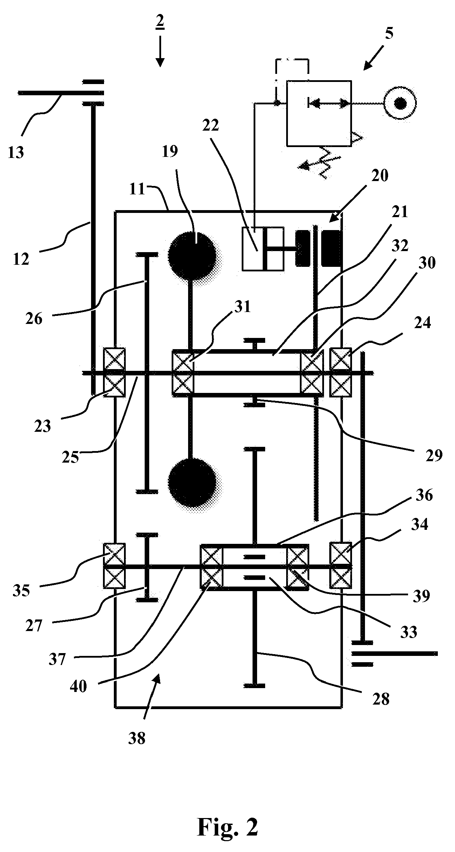

Figur 2 zeigt schematisch eine Ausführungsform eines Ergometers, wie es vorteilhaft in der in Figur 1 dargestellten Magnetkardiographieanordnung als Belastungseinheit 2 eingesetzt werden kann. Das Ergometer 2 ist als Fahrradergometer ausgestaltet und größtenteils aus Polyoxymethylen (POM) und einem oder mehreren anderen nicht-ferromagnetischen Kunststoffen/Metallen/Keramiken gefertigt. In einem Gehäuse 11 ist eine Schwungmasse 19 angeordnet, die beispielsweise aus POM oder Aluminium gefertigt ist und über ein zweistufiges Getriebe 38 in Rotation versetzt werden kann. Hierzu sind mittels Lagern 23, 24 und 34, 35 zwei parallel zueinander angeordnete Wellen 25 und 37 drehbar in dem Gehäuse 11 gelagert. Die hier als Vollwelle ausgebildete erste Welle 25 ist über Öffnungen im Gehäuse 11 nach außen geführt. Dort sind Pedalarme 12 mit Pedalen 13 an der Welle 25 montiert. Mit Hilfe der Pedalarme 12 und Pedalen 13 kann die Welle 25 in Rotation versetzt werden. Im Inneren des Gehäuses 11 ist auf der Welle 25 ein Riemenrad 26 fixiert, das mit der Welle 25 in Rotation versetzt wird. Dem Riemenrad 26 auf der Welle 25 ist ein Riemenrad 27 auf der ebenfalls als Vollwelle ausgestalteten zweiten Welle 37 zugeordnet. Über einen hier nicht dargestellten über die Umfangsflächen der Riemenräder 26, 27 geführten Riemen wird bei Rotation der ersten Welle 25 auch die zweite Welle 37 in Rotation versetzt. Das Riemenrad 26 weist einen größeren Durchmesser auf als das Riemenrad 27, so dass ein entsprechendes erstes Übersetzungsverhältnis resultiert. Außen auf der Welle 37 ist koaxial zur Welle 37 mittels Lagern 39, 40 eine als Hohlwelle ausgeführte dritte Welle 36 drehbar gelagert, in der eine Freilaufkupplung 33 angeordnet ist. Auf der Hohlwelle 36 ist ein Riemenrad 28 befestigt. Die Freilaufkupplung 33 ist so eingerichtet, dass die Hohlwelle 36 und das Riemenrad 28 bei vorgesehener Rotationsrichtung mit der Welle 37 rotieren, bei entgegengesetzter Rotationsrichtung oder bei langsamerer Rotation der Welle 37 gegenüber der Hohlwelle 36 eine Entkopplung der Hohlwelle 36 gegenüber der Welle 37 erfolgt. Dem Riemenrad 28 ist ein Riemenrad 29 zugeordnet, das auf einer ebenfalls als Hohlwelle ausgeführten vierten Welle 32 montiert ist, die mittels Lagern 30, 31 auf der Welle 25 gelagert ist. Ein hier nicht dargestellter um die Umfangsflächen der Riemenräder 28, 29 gelegter Riemen überträgt die Rotationsbewegung der Hohlwelle 36 auf die Hohlwelle 32. Das Riemenrad 28 weist hier einen größeren Durchmesser auf als das Riemenrad 29, so dass ein entsprechendes zweites Übersetzungsverhältnis resultiert. Über die Verhältnisse der Durchmesser der Riemenräder 26, 27, 28 und 29 zueinander können die jeweiligen Übersetzungsverhältnisse eingestellt und auf die jeweiligen Bedürfnisse abgestimmt werden. Insgesamt ergibt sich zwischen der ersten Welle 25 und der vierten Welle 32 ein Übersetzungsverhältnis von mindestens 1:10, beispielsweise von 12,25. Auf der Hohlwelle 32 ist eine beispielsweise scheibenförmige Schwungmasse 19 befestigt, die mit der Hohlwelle 32 rotiert. Auf der Hohlwelle 32 ist auch eine Bremsscheibe 21 befestigt, die mit einem Pneumatikzylinder 22 so in Eingriff gebracht werden kann, dass die Reibung zwischen der Bremsscheibe 21 und dem Pneumatikzylinder 22 der Rotationsbewegung der Hohlwelle 32 und damit der Schwungmasse 19 einen entsprechenden Widerstand entgegensetzt. Der Anpressdruck des Pneumatikzylinders 22 auf der Bremsscheibe 21 und damit der Widerstand gegen die Rotationsbewegung der Schwungmasse 19 kann mittels einer Druckregelungseinheit 5 vorzugsweise stufenlos eingestellt werden.

Wenn das Ergometer 2 in der richtigen Richtung betätigt wird, beispielsweise durch vorwärts gerichtete Tretbewegungen eines Patienten, wird somit über die Pedalarme 12 zunächst die Welle 25 mit dem darauf befindlichen Riemenrad 26 in Rotation versetzt. Diese Rotationsbewegung wird über einen Riemen zwischen dem Riemenrad 26 und dem Riemenrad 27 mit einem ersten Übersetzungsverhältnis auf die in dem Gehäuse 11 drehbar gelagerte zur Welle 25 parallelen Welle 37 übertragen. Die Freilaufkupplung 33 bringt die Hohlwelle 36 mit der Welle 37 in Eingriff, so dass die Hohlwelle 36 mit der Welle 37 rotiert. Das dadurch ebenfalls in Rotation versetzte Riemenrad 28 überträgt die Rotationsbewegung über einen Riemen, der das Riemenrad 28 mit dem Riemenrad 29 verbindet, mit einem zweiten Übersetzungsverhältnis auf die drehbar auf der Welle 25 gelagerten Hohlwelle 32. Dadurch wird die auf der Hohlwelle 32 angeordnete Schwungmasse 19 in Rotation versetzt. Verlangsamt sich die Rotationsbewegung der Welle 25 und damit der Welle 37 oder stoppt die Rotationsbewegung ganz, beispielsweise, weil der Patient langsamer tritt oder ganz mit der Tretbewegung aufhört, sorgt die zwischen der ersten und zweiten Übersetzungsstufe angeordnete Freilaufkupplung 33 dafür, dass die Pedalarme 12 nicht weiter rotieren, während die Schwungmasse 19 ihre Rotationsbewegung um die Welle 25 fortsetzt, sofern sie nicht durch den auf die Bremsscheibe 21 einwirkenden Pneumatikzylinder 22 abgebremst bzw. gestoppt wird.

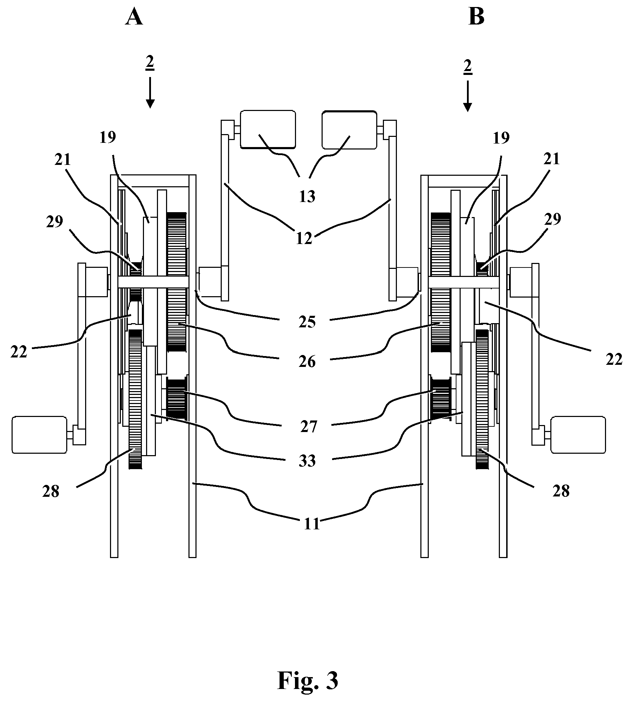

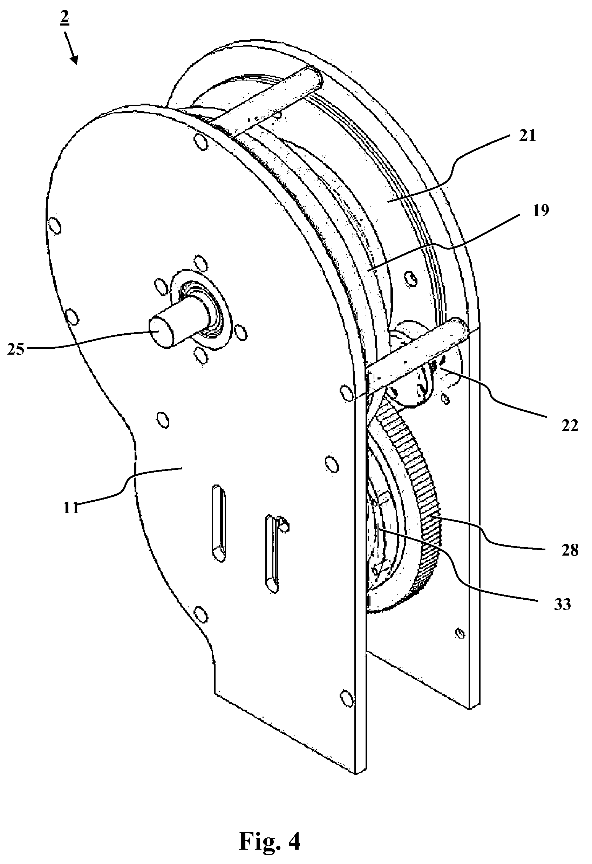

Die Figuren 3 und 4 zeigen Ansichten einer Ausführungsform des Ergometers. Figur 3A zeigt eine Frontansicht, Figur 3B eine Rückansicht und Figur 4 eine isometrische Ansicht, wobei ein Teil des Gehäuses 11 jeweils entfernt wurde. Für eine Beschreibung der Funktion und des Zusammenwirkens der einzelnen Ergometerkomponenten wird zur Vermeidung von Wiederholungen auf Figur 2 verwiesen. Hier ist zusätzlich erkennbar, dass die Riemenräder 26, 27, 28, 29 vorzugsweise als Zahnriemenräder ausgeführt sind. Darüber hinaus ist ersichtlich, dass die Schwungmasse 19 scheibenförmig ausgestaltet ist. Das Ergometer weist eine sehr kompakte Anordnung der Einzelkomponenten auf.

### Bezugszeichenliste:

- 1: Magnetkardiograph
- 2: Belastungseinheit
- 3: Patientenliege
- 4: Abschirmungskammer
- 5: Druckregelungseinheit
- 6: Quelle für ein druckbeaufschlagtes Gas oder eine druckbeaufschlagte Gasmischung
- 7: Bedien- und Steuereinheit
- 8: Leitungen
- 9: Überwachungskamera
- 10: Versorgungs- und Signalleitungen
- 11: Gehäuse
- 12: Pedalarm
- 13: Pedalen
- 14: Signalleitung
- 15: Leitungen
- 16: Durchführung
- 17: Durchführung
- 18: Durchführung
- 19: Schwungmasse
- 20: Bremseinheit
- 21: Bremsscheibe
- 22: Pneumatikzylinder
- 23: Lager
- 24: Lager
- 25: Welle
- 26: Riemenrad
- 27: Riemenrad
- 28: Riemenrad
- 29: Riemenrad
- 30: Lager
- 31: Lager
- 32: Welle
- 33: Freilaufkupplung
- 34: Lager
- 35: Lager
- 36: Welle
- 37: Welle
- 38: Getriebe
- 39: Lager
- 40: Lager

## Patentansprüche

1. Magnetkardiographieanordnung zur Erstellung von Belastungs-Magnetkardiogrammen, umfassend einen Magnetkardiographen (1) und eine aus nicht-magnetischen Komponenten bestehende Belastungseinheit (2), wobei der Magnetkardiograph (1) und die Belastungseinheit (2) in einer Abschirmkammer (4) zur Abschirmung externer Magnetfelder angeordnet sind, wobei die Belastungseinheit (2) ein mittels druckbeaufschlagtem Gas oder druckbeaufschlagter Gasmischung aus einer außerhalb der Abschirmkammer (4) angeordneten Quelle (6) für das druckbeaufschlagte Gas oder die druckbeaufschlagte Gasmischung gebremstes Fahrrad-Ergometer ist oder umfasst, und die Belastungseinheit (2) eine mittels Hand- oder Fußbetätigung über ein Getriebe (38) mit einem Übersetzungsverhältnis von mindestens 1:10 in Rotation versetzbare Schwungmasse (19) und eine mittels des druckbeaufschlagten Gases oder der druckbeaufschlagten Gasmischung betätigbare Bremseinheit (20) zur Abbremsung der Rotation der Schwungmasse (19) umfasst, wobei die Bremskraft der Bremseinheit (20) mittels des druckbeaufschlagten Gases oder der druckbeaufschlagten Gasmischung einstellbar ist.

2. Magnetkardiographieanordnung nach Anspruch 1, wobei es sich bei dem druckbeaufschlagten Gas oder der druckbeaufschlagten Gasmischung um Druckluft, Stickstoff, Sauerstoff, Helium, Sauerstoff oder Mischungen davon, vorzugsweise um Druckluft, handelt.

3. Magnetkardiographieanordnung nach Anspruch 1 oder 2, wobei das Getriebe (38) mindestens zweistufig und das Übersetzungsverhältnis 1:10 bis 1:20, bevorzugt 1:10 bis 1:18, weiter bevorzugt 1:10 bis 1:15 und besonders bevorzugt 1:10 bis 1:13 beträgt.

4. Magnetkardiographieanordnung nach Anspruch 3, wobei das Getriebe (38) der Belastungseinheit (2) vier Wellen (25, 37, 36, 32) umfasst, von denen die erste und zweite Welle (25, 37) bevorzugt jeweils als Vollwelle und die dritte und vierte Welle (36, 32) jeweils als Hohlwelle ausgebildet sind, und wobei die vierte Welle (32) koaxial drehbar auf der ersten Welle (25) gelagert ist, und die dritte Welle (36) koaxial drehbar auf der zweiten Welle (37) gelagert ist, die Schwungmasse (19) auf der vierten Welle (32) befestigt ist, und wobei die Wellen (25, 37, 36, 32) derart in Eingriff gebracht sind, dass eine Rotationsbewegung der ersten Welle (25) mit einem ersten Übersetzungsverhältnis auf die zweite Welle (37), von dieser auf die dritte Welle (36) und von dieser mit einem zweiten Übersetzungsverhältnis auf die vierte Welle (32) übertragen wird.

5. Magnetkardiographieanordnung nach Anspruch 4, wobei zwischen der zweiten Welle (37) und der dritten Welle (36) eine Freilaufkupplung (33) angeordnet ist.

6. Magnetkardiographieanordnung nach einem der Ansprüche 3 bis 5, wobei das Getriebe (38) ein Riemengetriebe ist.

7. Magnetkardiographieanordnung nach Anspruch 6, wobei auf der ersten Welle (25) ein erstes Riemenrad (26), auf der zweiten Welle (37) ein zweites Riemenrad (27), auf der dritten Welle (36) ein drittes Riemenrad (28) und auf der vierten Welle (32) ein viertes Riemenrad (29) angeordnet ist, und wobei das erste und zweite Riemenrad (26, 27) sowie das dritte und vierte Riemenrad (28, 29) jeweils über einen Riemen verbunden sind.

8. Magnetkardiographieanordnung nach einem der Ansprüche 3 bis 7, wobei die Bremseinheit (20) einen Pneumatikzylinder (22) umfasst, der mit einer auf der vierten Welle (32) befestigten Bremsscheibe (21) in Eingriff gebracht werden kann.

9. Magnetkardiographieanordnung nach einem der vorhergehenden Ansprüche, wobei die Belastungseinheit (2) an einer aus nicht-magnetischen Komponenten bestehenden Patientenliege (3) angeordnet ist.

10. Magnetkardiographieanordnung nach einem der vorhergehenden Ansprüche, wobei die Belastungseinheit (2) mittels nicht-magnetischer Leitungen (8) mit einer vorzugsweise innerhalb der Abschirmkammer (4) angeordneten Druckregelungseinheit (5) verbunden ist und die Druckregelungseinheit (5) mit der außerhalb der Abschirmkammer (4) angeordneten Quelle (6) für das druckbeaufschlagte Gas oder die druckbeaufschlagte Gasmischung, die vorzugsweise eine Druckluftquelle ist, verbunden ist.

11. Magnetkardiographieanordnung nach Anspruch 10, **dadurch gekennzeichnet, dass** der Magnetkardiograph (1) und/oder die Druckregelungseinheit (5) mit einer außerhalb der Abschirmkammer (4) angeordneten Bedien- und Steuereinheit (7) verbunden ist/sind.

12. Magnetkardiographieanordnung nach Anspruch 11, **dadurch gekennzeichnet, dass** der mittels der Druckregelungseinheit (5) eingestellte Widerstand für die Belastungseinheit (2) über die Bedien- und Steuereinheit (7) mess- und/oder regelbar ist.

13. Magnetkardiographieanordnung nach einem der Ansprüche 10 bis 12, **dadurch gekennzeichnet, dass** zur Parallel-Aufzeichnung eines EKG ein außerhalb der Abschirmkammer (4) positioniertes EKG-Gerät vorgesehen ist, das aus nicht-magnetischen Komponenten bestehende, in die Abschirmkammer (4) reichende, mit dem Patienten verbindbare EKG-Elektrodenstecker und -Ableitungen aufweist.

## Claims

1. A magnetocardiography arrangement for producing stress magnetocardiograms, comprising a magnetocardiograph (1) and a stress unit (2) consisting of non-magnetic components, wherein the magnetocardiograph (1) and the stress unit (2) are arranged in a shielded chamber (4) for shielding against external magnetic fields, wherein the stress unit (2) is or comprises a bicycle ergometer braked by means of pressurised gas or a pressurised gas mixture from a source (6) for the pressurised gas or the pressurised gas mixture arranged outside the shielded chamber (4), and wherein the stress unit (2) comprises a flywheel mass (19) rotatable by means of hand or foot actuation via a transmission (38) with a gear ratio of at least 1:10 and a brake unit (20) actuatable by means of the pressurised gas or the pressurised gas mixture for braking the rotation of the flywheel mass (19), wherein the braking force of the brake unit (20) is adjustable by means of the pressurised gas or the pressurised gas mixture.

2. The magnetocardiography arrangement according to claim 1, wherein the pressurised gas or the pressurised gas mixture is compressed air, nitrogen, oxygen, helium, oxygen or mixtures thereof, preferably compressed air.

3. The magnetocardiography arrangement according to claim 1 or 2, wherein the transmission (38) is a at least two-stage transmission (38) and the gear ratio is 1:10 to 1:20, more preferably 1:10 to 1:18, more preferably 1:10 to 1:15 and particularly preferably 1:10 to 1:13.

4. The magnetocardiography arrangement according to claim 3, wherein the transmission (38) of the stress unit (2) comprises four shafts (25, 37, 36, 32), of which the first and second shaft (25, 37) are preferably each formed as solid shafts and the third and fourth shaft (36, 32) are preferably each formed as hollow shafts, and wherein the fourth shaft (32) is mounted coaxially rotatable on the first shaft (25), and the third shaft (36) is mounted coaxially rotatable on the second shaft (37), the flywheel mass (19) is fastened on the fourth shaft (32), and wherein the shafts (25, 37, 36, 32) are brought into engagement in such a way that a rotational movement of the first shaft (25) is transmitted with a first gear ratio to the second shaft (37), from this to the third shaft (36) and from this with a second gear ratio to the fourth shaft (32).

5. The magnetocardiography arrangement according to claim 4, wherein a freewheel clutch (33) is arranged between the second shaft (37) and the third shaft (36).

6. The magnetocardiography arrangement according to one of claims 3 to 5, wherein the transmission (38) is a belt drive.

7. The magnetocardiography arrangement according to claim 6, wherein a first belt pulley (26) is arranged on the first shaft (25), a second belt pulley (27) is arranged on the second shaft (37), a third belt pulley (28) is arranged on the third shaft (36), a fourth belt pulley (29) is arranged on the fourth shaft (32), and wherein the first and second belt pulley (26, 27) and also the third and fourth belt pulley (28, 29) are connected in each case via a belt.

8. The magnetocardiography arrangement according to one of claims 3 bis 7, wherein the brake unit (20) comprises a pneumatic cylinder (22), which can be brought into engagement with a brake disc (21) fastened on the fourth shaft (32).

9. The magnetocardiography arrangement according to one of the preceding claims, wherein the stress unit (2) is arranged on a patient bed (3) consisting of non-magnetic components.

10. The magnetocardiography arrangement according to one of the preceding claims, wherein the stress unit (2) is connected by means of non-magnetic lines (8) to a pressure regulation unit (5) preferably arranged within the shielded chamber (4) and the pressure regulation unit (5) is connected to the source (6), arranged outside the shielded chamber (4), for the pressurised gas or the pressurised gas mixture, which is preferably a compressed air source.

11. The magnetocardiography arrangement according to claim 10, **characterised in that** the magnetocardiograph (1) and/or the pressure regulation unit (5) is/are connected to an operating and control unit (7) arranged outside the shielded chamber (4).

12. The magnetocardiography arrangement according to claim 11, **characterised in that** the resistance for the stress unit (2) set by means of the pressure regulation unit (5) can be measured and/or regulated via the operating and control unit (7).

13. The magnetocardiography arrangement according to one of claims 10 to 12, **characterised in that**, to record an ECG in parallel, an ECG device positioned outside the shielded chamber (4) is provided, consisting of non-magnetic components and having ECG electrode plugs and leads, reaching into the shielded chamber (4) and connectable to the patient.

## Revendications

1. Système de magnétocardiographie pour l'établissement de magnétocardiogrammes d'effort comprenant un magnétocardiographe (1) et une unité de sollicitation (2) constituée de composants non magnétiques pour lequel le magnétocardiographe (1) et l'unité de sollicitation (2) sont disposés dans une chambre de blindage (4) pour la protection contre les champs magnétiques externes, pour lequel l'unité de sollicitation (2) est ou comprend un vélo ergomètre freiné au moyen d'un gaz sous pression ou d'un mélange de gaz sous pression à partir d'une source (6) disposée en dehors de la chambre de blindage (4) pour le gaz sous pression ou le mélange de gaz sous pression et l'unité de sollicitation (2) comprend une masse d'inertie (19) pouvant être mise en rotation au moyen d'une commande à main ou à pied par un mécanisme (38) avec un rapport de transmission d'au moins 1 :10 et une unité de freinage (20) pouvant être actionnée au moyen du gaz sous pression ou du mélange de gaz sous pression pour freiner la rotation de la masse d'inertie (19), pour lequel la force de freinage de l'unité de freinage (20) peut être réglée au moyen du gaz sous pression ou du mélange de gaz sous pression.

2. Système de magnétocardiographie selon la revendication 1, pour lequel il s'agit concernant le gaz sous pression ou le mélange de gaz sous pression, d'air comprimé, d'azote, d'oxygène, d'hélium, d'oxygène ou mélanges de celui-ci, de préférence d'air comprimé.

3. Système de magnétocardiographie selon la revendication 1 ou 2, pour lequel le mécanisme (38) comporte au moins deux niveaux et le rapport de transmission est de 1 :10 à 1 :20, de préférence 1 :10 à 1 :18, de préférence encore 1 :10 à 1:15 et de préférence en particulier de 1 :10 à 1 :13.

4. Système de magnétocardiographie selon la revendication 3, pour lequel le mécanisme (38) de l'unité de sollicitation (2) comprend quatre arbres (25, 37, 36, 32), dont le premier et deuxième arbre (25, 37) sont constitués de préférence respectivement comme arbres pleins et le troisième et quatrième arbre (36, 32) respectivement comme arbres creux et pour lequel le quatrième arbre (32) est logé coaxialement pouvant tourner sur le premier arbre (25) et le troisième arbre (36) est logé coaxialement pouvant tourner sur le deuxième arbre (37), la masse d'inertie (19) est fixée sur le quatrième arbre (32) et pour lequel les arbres (25, 37, 36, 32) sont mis en prise de telle sorte qu'un mouvement de rotation du premier arbre (25) est transmis avec un premier rapport de transmission au deuxième arbre (37), de celui-ci au troisième arbre (36) et de celui-ci au quatrième arbre (32) avec un deuxième rapport de transmission.

5. Système de magnétocardiographie selon la revendication 4, pour lequel un accouplement à roue libre (33) est disposé entre le deuxième arbre (37) et le troisième arbre (36).

6. Système de magnétocardiographie selon l'une quelconque des revendications 3 à 5, pour lequel le mécanisme (38) est un mécanisme à courroies.

7. Système de magnétocardiographie selon la revendication 6, pour lequel sur le premier arbre (25) est située une première roue à courroies (26), sur le deuxième arbre (37) une deuxième roue à courroie (27), sur le troisième arbre (36) une troisième roue à courroie (28) et sur le quatrième arbre (32) une quatrième roue à courroie (29) et pour lequel la première et deuxième roue à courroie (26, 27) ainsi que la troisième et quatrième roue à courroie (28, 29) sont respectivement reliées par une courroie.

8. Système de magnétocardiographie selon l'une quelconque des revendications 3 à 7, pour lequel l'unité de freinage (20) comprend un vérin pneumatique (22) qui peut être mis en prise avec un disque de frein (21) fixé sur le quatrième arbre (32).

9. Système de magnétocardiographie selon l'une quelconque des revendications précédentes, pour lequel l'unité de freinage (2) est disposée sur un lit de patient (3) constitué de composants non magnétiques.

10. Système de magnétocardiographie selon l'une quelconque des revendications précédentes, pour lequel l'unité de sollicitation (2) est raccordée au moyen de conduits non magnétiques (8) à une unité de régulation de pression (5) disposée de préférence à l'intérieur de la chambre de blindage (4) et l'unité de régulation de pression (5) est raccordée à la source (6) disposée en dehors de la chambre de blindage (4) pour le gaz sous pression ou le mélange de gaz sous pression, qui est de préférence une source d'air comprimé.

11. Système de magnétocardiographie selon la revendication 10, **caractérisé en ce que** le magnétocardiographe (1) et/ou l'unité de régulation de pression (5) est/sont relié(s) à une unité de commande et de pilotage (7) disposée en dehors de la chambre de blindage (4).

12. Système de magnétocardiographie selon la revendication 11, **caractérisé en ce que** la résistance établie au moyen de l'unité de régulation de pression (5) pour l'unité de sollicitation (2) peut être mesurée et/ou réglée par l'unité de commande et de pilotage (7).

13. Système de magnétocardiographie selon l'une quelconque des revendications 10 à 12, **caractérisé en ce qu'**un appareil d'électrocardiogramme positionné en dehors de la chambre de blindage (4) pour l'enregistrement parallèle d'un ECG est prévu, qui comporte des connexions à électrodes et dérivations d'ECG, parvenant à la chambre de blindage (4), constituées de composants non magnétiques, pouvant être reliées au patient.
